# EUROPÄISCHE PATENTSCHRIFT

(11) **EP 0 702 674 B1**
(45) Veröffentlichungstag und Bekanntmachung des Hinweises auf die Patenterteilung: **18.08.2004**
(21) Anmeldenummer: 94920441.6
(22) Anmeldetag: 10.06.1994
(51) Int. Cl.: C07D 233/54, C07K 1/06

(54) **VERFAHREN ZUR PEPTIDSYNTHESE UNTER VERWENDUNG VON SCHUTZ- UND ANKERGRUPPEN**
PROCESS FOR THE SYNTHESIS OF PEPTIDES USING PROTECTING AND ANCHOR GROUPS
PROCEDE POUR LA SYNTHESE DE PEPTIDES UTILISANTS DES GROUPES PROTECTEURS ET D'ANCRAGE

(30) Priorität: 11.06.1993 DE 4319475; 18.06.1993 DE 4320260
(43) Veröffentlichungstag der Anmeldung: 27.03.1996
(60) Teilanmeldung: 02027593.9
(73) Patentinhaber: Gesellschaft für Biotechnologische Forschung mbH (GBF), 38124 Braunschweig (DE)
(72) Erfinder: FRANK, Ronald, D-38124 Braunschweig (DE); HOFFMANN, Stefan, D-38124 Braunschweig (DE)
(74) Vertreter: Boeters, Hans Dietrich, Dr.
(86) Internationale Anmeldenummer: PCT/EP1994/001896
(87) Internationale Veröffentlichungsnummer: WO 1994/029278

(56) Entgegenhaltungen:
- EP-A- 0 061 933
- EP-A- 0 322 348
- WO-A-83/02448
- WO-A-94/01409
- FR-A- 1 472 013
- TETRAHEDRON LETTERS, Bd.28, Nr.46, 1987, OXFORD GB Seiten 5651 - 5654 G. BREIPOHL ET AL. 'Preparation and application of new acid labile anchor groups for the synthesis of peptide amides by FMOC solid phase synthesis' in der Anmeldung erwähnt
- TETRAHEDRON LETTERS, Bd.30, Nr.20, 1989, OXFORD GB Seiten 2641 - 2644 F. GUIBE ET AL. 'Use of an allylic anchor group and its palladium catalyzed hydrostannolytic cleavage in the solid phase synthesis of protected peptide fragments' in der Anmeldung erwähnt
- Int. J. Peptide Chemistry, Protein Res. 42,1993, M. Patek, pages 97-117
- JERRY MARCH: "Advanced Organic Chemistry", 1985, JOHN WILEY AND SONS, NEW YORK

## Beschreibung

Die regioselektive chemische Umsetzung einer Verbindung mit mehreren auch unterschiedlichen reaktiven chemischen Funktionen erfordert den Schutz aller dieser Funktionen bis auf diejenige(n), mit der (denen) eine chemische Reaktion eingegangen werden soll. Zum Schutz dieser Funktionen werden Molekülgruppen (Schutzgruppen) eingeführt. Diese lassen sich im Anschluß schonend und selektiv unter Zurückbildung der ursprünglichen Funktion abspalten. Für komplexe und mehrstufige Synthesen, insbesondere von Naturstoffen, wie Oligopeptiden und Oligonucleotiden, sind verschiedene Typen von Schutzgruppen notwendig. Sie zeichnen sich durch stark unterschiedliche Bedingungen der Abspaltung aus. Ein System von Schutzgruppen, in dem die einzelnen Typen so selektiv spaltbar sind, daß jeweils alle anderen Schutzgruppen unberührt bleiben, wird orthogonal genannt. Der Fachmann ist mit dem Prinzip der Schutzgruppenchemie vertraut. Verwiesen sei auf die im folgenden angeführte Literatur. Weist der Schutzgruppentyp noch eine weitere reaktive Funktion auf, so daß er kovalent und stabil mit einem Trägermaterial für die Festphasensynthese verknüpft werden kann, dann spricht der Fachmann von einer "Ankergruppe" oder "Linkergruppe". Ein spezieller Typ von Schutzgruppe liegt dann vor, wenn die Schutzgruppe erst durch eine der Abspaltung vorgeschaltete chemische Reaktion in eine labile Form gebracht werden muß, die dann in einem zweiten Schritt unter milden Bedingungen abgespalten werden kann. Man spricht in diesem Fall von einer geschützten Schutzgruppe beziehungsweise "safety-catch group". Obwohl hier zwei Reaktionsschritte zur Abspaltung notwending sind, kann eine derartige Gruppe doch große Vorteile bieten:
- sie kann sehr stabil gegen viele, auch sehr drastische Reaktionsbedingungen sein, jedoch durch die Folge von zwei spezifischen, sehr milden Reaktionsschritten abgespalten werden können;
- die labile Zwischenstufe der Schutzgruppe kann stabil genug sein, daß sie gute oder bessere Möglichkeiten zur Isolierung und Reinigung des Endproduktes bietet.

Aufgabe der vorliegenden Erfindung ist es, eine Peptidsynthese vorzusehen, mit der sich eine Carboxyl-Funktion (COOH) unter Ausbildung einer Esterbindung umsetzen läßt, wodurch eine Schutzgruppe und insbesondere eine Ankergruppe für die Carboxyl-Funktion vorgesehen werden kann, die folgende Merkmale aufweist:
- die Schutz- beziehungsweise Ankergruppe soll ihrerseits geschützt sein und sich in eine labile Zwischenstufe überführen lassen;
- die labile Zwischenstufe soll unter vorgegebenen Reaktionsbedingungen, und zwar denen einer Festphasensynthese von Peptiden, stabil sein und eine Reinigung der Zwischenprodukte erlauben;
- die labile Zwischenstufe soll in wässriger physiologischer Pufferlösung, vorzugsweise bei neutralem pH (etwa pH 7) oder nahezu neutralem pH (5 bis 9), zerfallen und die ursprüngliche Carboxyl-Funktion zurückbilden können, so daß das Syntheseprodukt mit freier Carboxyl-Funktion direkt (ohne weitere Reinigung) in ein zellbiologisches oder biochemisches Testexperiment einsetzbar ist.

So soll dieser Schutzgruppentyp als Ankergruppe für die Festphasensynthese von Peptiden einsetzbar sein, insbesondere nach der Fmoc/tBu-Methode; vergleiche beispielsweise Fields & Noble in Int. J. Peptide Protein Res., 35 (1990) 161-214.

So wird die der Erfindung zugrunde liegende Aufgabe durch eine Peptidsynthese gelöst, bei der man
(i) eine Verbindung der Formel

   Y-C(R₂) (R₃)-B-X

   wobei
   Y = HO, Cl, Br oder I,
   B = aromatischer stickstoffhaltiger Fünfring,
   X = Schutzgruppe für B, die die Basizität von B soweit herabsetzt, daß die intramolekulare Hydrolyse der Esterbindung erst nach Abspaltung von X erfolgen kann,
   R₂ = H, Alkyl oder Aryl, und
   R₃ = HOOC; oder Alkyl oder Aryl, jeweils durch eine reaktive Funktion substituiert, die eine Ankergruppe bildet,
   mit einem Trägermaterial verknüpft,
(ii) danach die mit dem Träger verknüpfte Verbindung mit einer Carboxyl-Funktion (COOH) unter Ausbildung einer Esterbindung eines Esters eines Mono-, Di- oder Oligopeptids umsetzt und ein Peptid synthetisiert,
(iii) danach die Schutzgruppe X selektiv abspaltet,
(iv) danach das angefallene Zwischenprodukt reinigt und
(v) danach das angefallene Zwischenprodukt in wässeriger Lösung zerfallen lässt und das Peptid als Syntheseprodukt mit freier Carboxyl-Funktion gewinnt.

In der vorstehenden Formel kann B = Imidazol-yl sein, insbesondere Imidazol-4-yl oder Imidazol-2-yl.

Ferner wird die der Erfindung zugrunde liegende Aufgabe durch eine Peptidsynthese gelöst, bei der man
(i) eine Verbindung der Formel

   Y-C(R₂) (R₃)-B-X

   wobei
   Y = HO, Cl, Br oder I,
   B = gegebenenfalls im Ring methylsubstituiertes Imidazol-yl oder gegebenenfalls im Ring methylsubstituiertes Imidazol-yl-methylen,
   X = Schutzgruppe für B, die die Basizität von B soweit herabsetzt, daß die intramolekulare Hydrolyse der Esterbindung erst nach Abspaltung von X erfolgen kann,
   R₂ = H, Alkyl oder Aryl, und
   R₃ = HOOC; oder Alkyl oder Aryl, jeweils durch eine reaktive Funktion substituiert, die eine Ankergruppe bildet,
   mit einem Trägermaterial verknüpft,
(ii) danach die verknüpfte Verbindung mit einer Carboxyl-Funktion (COOH) unter Ausbildung einer Esterbindung eines Esters eines Mono-, Di- oder Oligopeptids umsetzt und ein Peptid synthetisiert,
(iii) danach die Schutzgruppe X selektiv abspaltet,
(iv) danach das angefallene Zwischenprodukt reinigt und
(v) danach das angefallene Zwischenprodukt in wässeriger Lösung zerfallen lässt und das Peptid als Syntheseprodukt mit freier Carboxyl-Funktion gewinnt.

In der vorstehenden Formel kann B = Imidazol-4-yl-methylen oder 5-Methyl-imidazol-4-yl sein.

Die erfindungsgemäße Peptidsynthese kann dadurch gekennzeichnet sein, dass man Stufe '(v) in physiologischer Pufferlösung durchführt.

Ferner kann die erfindungsgemäße Peptidsynthese dadurch gekennzeichnet sein, dass man die Peptidsynthese bei Stufe (ii) unter basischen Reaktionsbedingungen durchführt.

Ferner kann die erfindungsgemäße Peptidsynthese dadurch gekennzeichnet sein, dass man in der Verbindung der vorstehenden Formel R₃ durch H₂N, HS, OH oder COOH substituiert ist.

Ferner kann die erfindungsgemäße Peptidsynthese dadurch gekennzeichnet sein, dass man eine Verbindung der vorstehenden Formel verwendet mit R₃ = HOOC-C₁₋₁₂-alkyl, HOOC-phenyl, HO-C₁₋₁₂-alkyl, HO-phenyl, H₂N-C₁₋₁₂-alkyl, H₂N-phenyl, HS-C₁₋₁₂alkyl oder HS-phenyl.

Ferner kann die erfindungsgemäße Peptidsynthese dadurch gekennzeichnet sein, daß man eine Verbindung der vorstehenden Formel verwendet mit X = Urethan- oder Alkoxycarbonyl Schutzgruppe, insbesondere mit X = tert-Butoxycarbonyl (Boc).

Ferner kann die erfindungsgemäße Peptidsynthese dadurch gekennzeichnet sein, daß man eine Verbindung der vorstehenden Formel verwendet mit R₂ = C₁₋₁₂-Alkyl oder Phenyl.

Erfindungsgemäß wird also eine Verbindung der Formel

Y-C(R₂) (R₃)-B-X

verwendet wobei die Verbindung mit einer Carboxyl-Funktion durch Substituieren der Y-Gruppe unter Ausbildung einer Esterbindung umgesetzt werden kann, wodurch eine Schutzgruppe und insbesondere eine Ankergruppe für die Carboxyl-Funktion vorgesehen wird, wobei
Y = HO, Cl, Br oder I,
B = basische Gruppe mit den angegebenen Bedeutungen ist, die durch X geschützt ist, nur hydrolyseempfindliche Acylverbindungen eingeht und entschützt intramolekular die Hydrolyse der Esterbindung katalysieren kann,
X = eine Schutzgruppe für die basische Gruppe B bedeutet und deren Katalysevermögen blockiert,
R₂ und R₃ = beliebige Reste mit den angegebenen Bedeutungen sind, die nicht die Bildung der Esterbindung und deren Hydrolyse beeinträchtigen.

Wie bereits gesagt, ist der Fachmann mit dem Prinzip der Schutzgruppenchemie vertraut. Zur Erläuterung sei auf folgenden Stand der Technik verwiesen.
Schutzgruppe (Protective Group): beispielsweise Greene & Wuts, Eds., Protective Groups in Organic Synthesis, 2nd Ed., 1991, John Wiley & Sons, NY.
Ankergruppe oder Linkergruppe: beispielsweise Breipohl et al. in Tetrahedron Lett., 28 (1987) 5651-5654; Guibe et al. in Tetrahedron Lett., 30 (1989) 2641-2644.
Geschützte Schutzgruppe (Safety-Catch Group): Patek in Int. J. Peptide Protein Res., 42 (1993) 97-117.
Elektronenziehende Schutzgruppe für eine basische Gruppe, wobei erstere die Basizität der basischen Gruppe herabsetzt: Gross & Meienhofer, Eds., The Peptides, Vol. 3, 1981, Academic Press, NY.; Patek in Int. J. Peptide Protein Res., 42 (1993) 97-117, insbesondere 112.

Die chemische Bindung zwischen Schutzgruppe und Carboxyl-Funktion soll also eine Esterbindung sein. Diese Esterbindung kann durch Umsetzung der Carboxyl-Funktion mit einer Hydroxyl-Funktion oder einer Halogen-Funktion bei der erfindungsgemäßen Peptidsynthese eingeführt werden. Carbonsäureester sind in wässriger Lösung mehr oder weniger leicht durch Katalyse von Basen hydrolytisch spaltbar. Die Schutzgruppe besitzt daher eine basische Funktion B, welche intramolekular die Hydrolyse der Esterbindung katalysieren kann. Diese basische Funktion B darf selbst keine hydrolysestabilen Acylverbindungen eingehen, da sonst die zu schützende Carboxylverbindung auf B übertragen werden könnte und somit keine hydrolytische Spaltung eintritt. Diese basische Funktion ist darüberhinaus durch eine Gruppe X geschützt, die die Basizität der Gruppe B soweit herabsetzt, daß eine intramolekular katalysierte Hydrolyse der Esterbindung erst nach Abspaltung der Gruppe X erfolgen kann. Die Gruppe X ist unter den Bedingungen einer an R₁ ansetzenden Synthese stabil.

Das folgende Reaktionsschema soll die erfindungsgemäße Peptidsynthese näher erläutern. Dabei bedeuten:
R₁ = Rest der zu schützenden Verbindung
R₂ und R₃ = Reste der Schutzgruppe mit den angegebenen Bedeutungen, welche nicht an deren Funktion teilhaben (R₃ weist eine zusätzliche reaktive Funktion für eine Verknüpfung mit Trägermaterialien auf, beispielsweise -COOH, -OH, -NH₂ oder -SH, und wird als Ankergruppe eingesetzt),
B = basische Funktion der angegebenen Bedeutungen und
X = Schutzgruppe der basischen Funktion der angegebenen Bedeutungen.

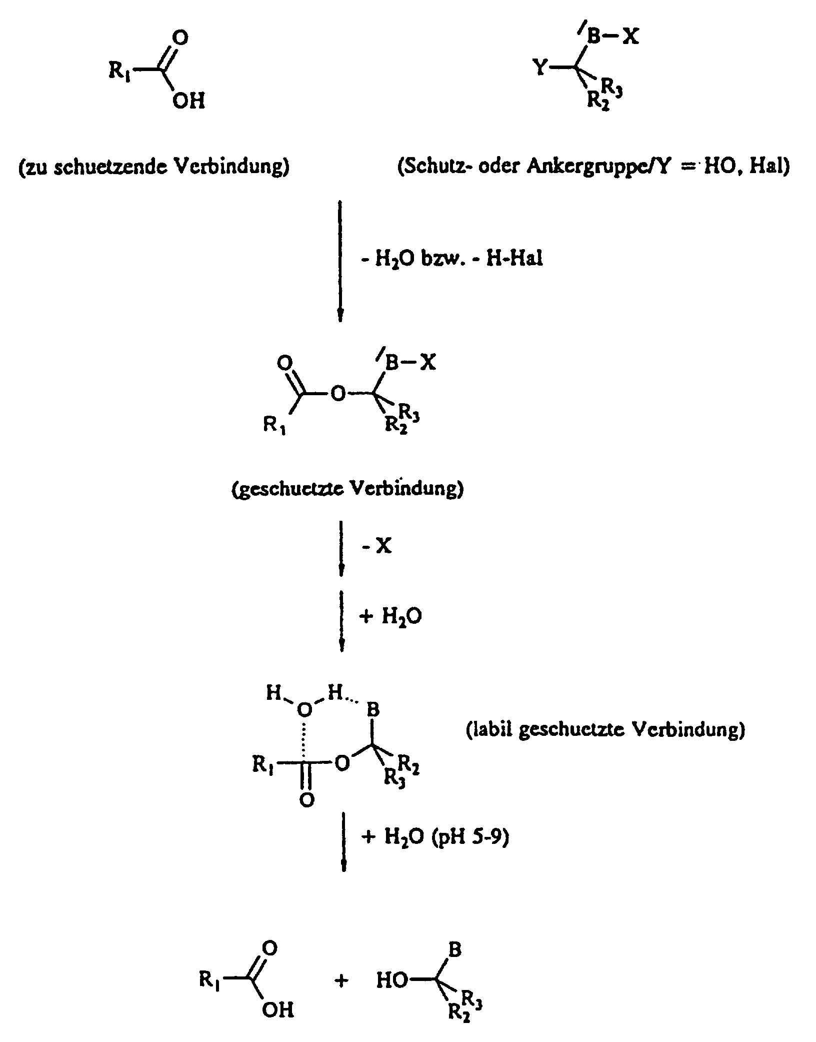

### Experimenteller Teil :

Alllgemeiner experimenteller Teil : Es wurden folgende analytisch-spektroskopische Apparaturen verwendet.- ¹H-NMR/¹³C-NMR : Bruker Modell AM-300 und WM-400 mit Tetramethylsilan (TMS) als interner Standard.- Signalmultiplizität : s = Singulett, d = dublett, t = Triplett, q = Quartett, ⁿJ_{H,H} = magnetische Kopplung über n Bindungen zwischen benachbarten Protonen.- FAB-MS : Kratos MS 50 TC RF mit neutralen Xenonstrahl (8-9 kV) und Finnigan Mat, Mass Spectrometer 8430 mit 3-Nitrobenzylalkohol als Matrix. Die Proben wurden in DMSO vorgelegt. UV/VIS : Carl Zeiss Modell PMQ II in Quarzküvetten mit 10 mm optischer Länge. ε(Dibenzofulven-Piperidin-Addukt/MeOH) = 5570. RP-C₁₈-HPLC : Analyt. HPLC : Pharmacia/LKB Pump P 3500, Liquid Chromatographie Controller LCC 500 Plus bzw. LKB 2249 Gradient Pump, LKB 2141 Variable Wavelength Monitor, Dreikanalflachbettschreiber auf Machery-Nagel Nucleosil 300-7 C₁₈ 250x4.

### Spezieller experimenteller Teil :

2-(1H-5-Methyl-imidazol-4-yl)-2-hydroxyessigsäure **(1)** : (C₆H₈N₂O₃) 18.25 g (0.17 mol) 4-Methyl-5-1H-imidazolcarbaldehyd [hergestellt nach Literaturvorschrift: Papadopoulos, E.P., Jarrar, A.und Issidorides, C.H., J. Chem. Soc. Chem. Comm. 615 (1966)]werden in 80 ml Wasser gelöst und bei 0°C unter Rühren 23.65 g (0.36 mol) Kaliumcyanid, 30 ml konz. Salzsäure (37 %) und 66 ml Acetanhydrid zugesetzt. Es wird 1 h bei 0°C gerührt und weitere 24 h bei RT. Es werden bei RT 15 ml konz. Salzsäure und 66 ml Acetanhydrid zugesetzt und weitere 24 h bei RT gerührt. Nach Zusetzen von 250 ml Wasser und 250 ml konz. Salzsäure wird unter Rückfluß auf 100°C erwärmt. Nach 2 h werden weitere 250 ml konz. Salzsäure zugegeben und 12 h bei 80°C gerührt. Die Lösung wird eingeengt und entweder durch präp. HPLC-Chromatographie auf Latek RP-C₁₈ HL 10µ 300x40 aufgereinigt (Ausb. 95 % d. Th.) oder **(1)** nach Stewart, C.P., J. Med. Chem. 130 (1923) über das Silbersalz gereinigt. Der Rückstand wird in Wasser gelöst und in salpetersaurer Lösung mit Sibernitrat gefällt. Nach Filtration der Lösung wird Bariumhydroxid zugesetzt und das Silbersalz von **(1)** zusammen mit überschüssigen Siberoxid abfiltriert. Der Filterkuchen wird in Wasser suspendiert und Schwefelwasserstoff eingeleitet. Nach Filtration der Lösung wird überschüssiges Barium durch vorsichtige Zugabe von Schwefelsäure gefällt. Nach erneuter Filtration wird die Lösung eingeengt und **(1)** durch mehrfache fraktionierende Kristallisation aus wässrigen Ethanol gewonnen.- Ausb. 42 % d. Th. 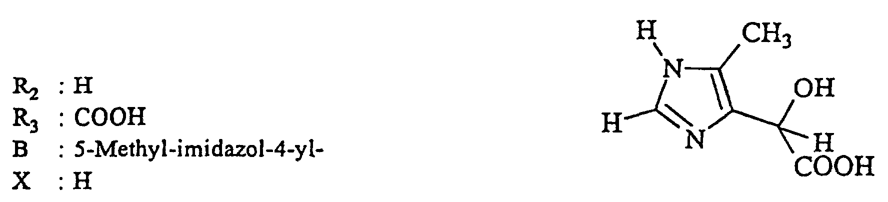 ¹H-NMR (400 MHz, D₂O) : δ = 8.57 (s, 1H, 2-H), 5.59 (s, 1H, CHOH), 2.33 (s, 3H, CH₃).-¹³C-NMR (75 MHz, D₂O) : δ = 174.0 (s, COOH), 135.2 (d, C-2), 132.3 (s, C-5), 119.9 (d, C-4), 65.5 (d, CHOH), 9.5 (q, CH₃).- MS (FAB) : M/Z (3-NBA) = 157 ([M+H]^{⊕}).

2-(1H-Imidazol-4-yl)-2-hydroxyessigsäure **(2)** : (C₅H₆N₂O₃) Die Synthese erfolgte analog zu **(1)** ausgehend von 4-1H-Imidazolcarbaldehyd.[hergestellt nach Literaturvorschrift: Weidenhagen, R. und Herrmann, R., Chem. Ber. 1955 (1953)] Ausb. 92 % d. Th. (nach RP-C₁₈-Chromatographie (Eluent : Wasser / 0.1 %TFA). Oder es wird wie unter **(1)** fraktioniert kristallisiert aus Wasser/Ethanol. 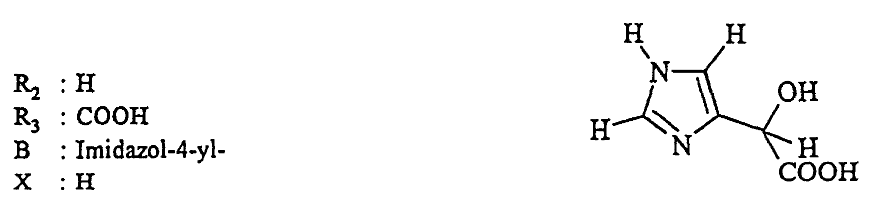 ¹H-NMR (400 MHz, D₂O) : δ = 8.70 (s, 1H, 2-H), 7.51 (s, 1H,4-H), 5.54 (s, 1H, CHOH).- ¹³C-NMR (75 MHz, D₂O) : δ = 173.5 (s, COOH), 135.0 (d, C-2), 131.2 (s, C-5), 117.9 (d, C-4), 65.2 (d, CHOH).- MS (FAB) : M/Z (3-NBA) = 143 ([M+H]^{⊕}).

2-(1H-Imidazol-2-yl)-2-hydroxyessigäure **(3)**: (C₅H₆N₂O₃) Die Synthese erfolgte analog zu **(1)** ausgehend von 2-1H-Imidazolcarbaldehyd [kommerziell erhältlich, z.B. Aldrich]. Ausb. 82 % d. Th. (RP-C₁₈-Chromatographie/Eluent Wasser / 0.1 %TFA). 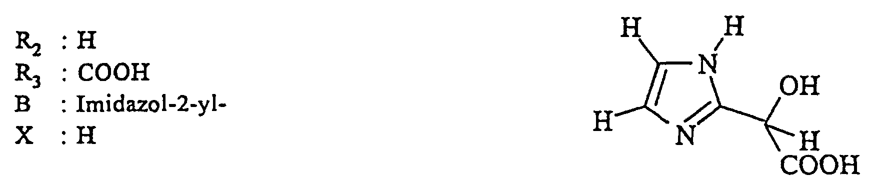 ¹H-NMR (400 MHz, D₂O) : δ = 7.38 (s, 2H, H-1), 5.69 (1H, s, H-2).- ¹³C-NMR (75 MHz, D₂O) : δ = 171.1 (s, COOH), 144.0 (s, C-2), 120.1 (d, C-4/C-5), 66.2 (d, CHOH).- MS (FAB) : M/Z (3-NBA) = 143 ([M+H]^{⊕}).

3-(N^{im}-tert-Butoxycarbonyl-imidazol-4-yl)-2-hydroxy-propionsäure **(4)** : (C₁₁H₁₆N₂O₅) 248 mg 3-1H-Imidazol-2-hydroxy-propionsäure (1.6 mmol) [kommerziell erhältlich, z.B. Sigma] werden in 2.5 ml abs. DMF unter Rühren bei 4°C suspendiert und 344 µl (2.4 mmol) tert-Butoxycarbonylazid zugegeben. Es wird 2 d bei 4°C gerührt und die Lösung bei 40°C am Rotationsverdampfer vollständig eingeengt. Der Rückstand wird in abs. Dioxan gelöst und der entstehende kristalline Niederschlag abfiltriert. Lyophilisation des Filtrats im HV ergibt einen farblosen, zähen Sirup. 356 mg (1.4 mmol), Ausb. 87 % d. Th. 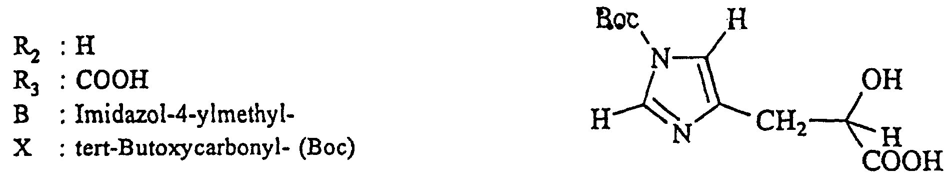 ¹H-NMR (300 MHz, CDCl₃) : δ = 8.15 (d, 1H, 2-H, ⁴J_{H,H}=1.25 Hz), 7.27 (s, 1H, 4-H), 4.39 (t, 1H, CHOH, ³J_{H,H}=4.5 Hz), 3.14 (AB-dd, 1H, -CH₂-, ²J_{H,H}=14.8 Hz, ³J_{H,H}=4.0 Hz), 3.02 (AB-dd, 1H, -CH₂-, ²J_{H,H}=14.8 Hz, ³J_{H,H}=5.0 Hz), 1.57 (s, 9H, CH₃).- ¹³C-NMR (75 MHz, CDCl₃) : δ = 176.1 (s,COOH), 146.3 (s, N-COO-), 136.8 (d, C-2), 135.9 (s, C-5), 116.1 (d, C-4), 86.4 (s, C(CH₃)₃), 68.5 (d, CHOH), 67.0 (t, -CH₂-), 27.8 (q, CH₃).- MS (FAB, DCHA-Derivat): M/Z (3-NBA) = 295 ([M+K]^{⊕}), 279 ([M+Na]^{⊕}), 257 ([M+H]^{⊕}), 201 ([M-57]^{⊕},-tBu), 182 ([DCHA+H]^{⊕}).

2-(N^{im}-tert-Butoxycarbonyl-imidazol-4-yl)-2-hydroxy-essigsäure **(5)** : (C₁₀H₁₄N₂O₅) Die Synthese erfolgte analog zu **(4)** ausgehend von **(2)**. Ausb. 52 % d. Th. 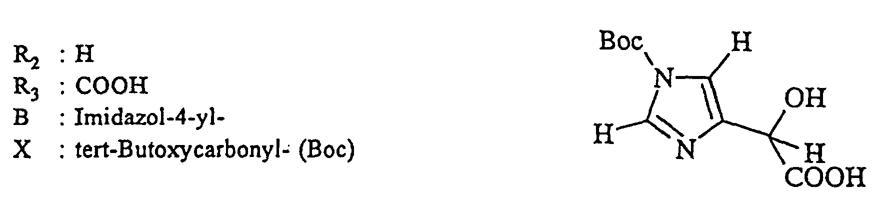 ¹H-NMR (400 MHz, CDCl₃) : δ = 8.15 (s, 1H, 2-H), 7.27 (s, 1H, 4-H), 5.14 (s, 1H, CHOH), 1.58 (s, 9H, CH₃).-¹³C-NMR (75 MHz, CDCl₃) : δ = 176.1 (s,COOH), 146.3 (s, N-COO-), 136.8 (d, C-2), 135.9 (s, C-5), 116.1 (d, C-4), 86.4 (s, C(CH₃)₃), 66.5 (d, CHOH), 27.8 (q, CH₃).

2-(N^{im}-tert-Butoxycarbonyl-5-methyl-imidazol-4-yl)-2-hydroxy-essigsäure **(6)** : (C₁₁H₁₆N₂O₅) Die Synthese erfolgte analog zu **(4)** ausgehend von **(1)** durch Umsetzung mit Pyrokohlensäure-di-tert-butylester oder tert-Butoxycabonylazid in DMF. Ausbeute 273 mg (1.1 mmol) 67 % d. Th. (weißes Lyophitisat aus Dioxan). 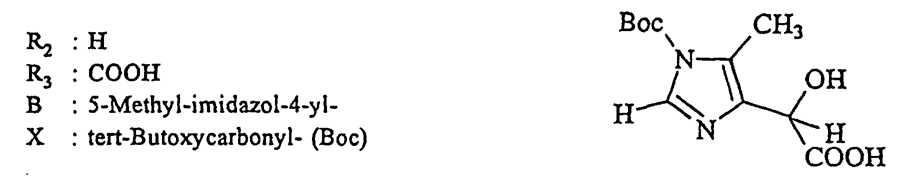 ¹H-NMR (400 MHz, CDCl₃) : δ = 8.15 (s, 1H, 2-H), 5.14 (s, 1H, CHOH), 2.46 (s, 3H, CH₃), 1.58 (s, 9H, CH₃).-¹³C-NMR (75 MHz, CDCl₃): δ = 174.6 (s, COOH), 147.1 (s, N-COO-), 137.0 (d, C-2), 136.2 (s, C-5), 127.2 (s, C-4), 86.4 (s, C(CH₃)₃), 66.5 (d, CHOH), 27.8 (q, CH₃).- MS (FAB): M/Z (3-NBA) = 279 ([M+Na]^{⊕}), 257 ([M+H]^{⊕}), 201 ([M-57+H]^{⊕}, tBu).

2-(N^{im}-tert-Butoxycarbonyl-imidazol-2-yl)-2-hydroxy-essigsäure **(7)** : (C₁₀H₁₄N₂O₅) Die Synthese erfolgte analog zu (**4**) ausgehend von (**3**) durch Umsetzung mit Pyrokohlensäure-di-tert-butylester in DMF. Ausb. 51 % d. Th.- ¹H-NMR (400 MHz, CDCl₃) : δ = 8.62 (s, 1H, 4-H), 7.27 (s, 1H, 5-H), 5.56 (s, 1H, CHOH), 1.58 (s, 9H, CH₃).

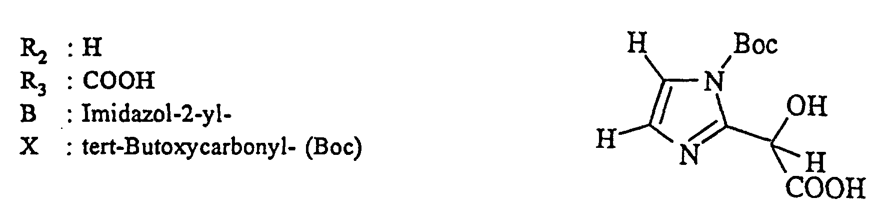

3-(1H-Imidazol-4-yl)-2-(phenylalanyl-phenylalanyl-glycyloxy)-propionyl-β-alanin **(8a)**: (C₂₉H₃₄N₆O₇)
MS (FAB) : M/Z (3-NBA) = 712 ([M+Cs+H]^{⊕}), 579 ([M+H]^{⊕}).

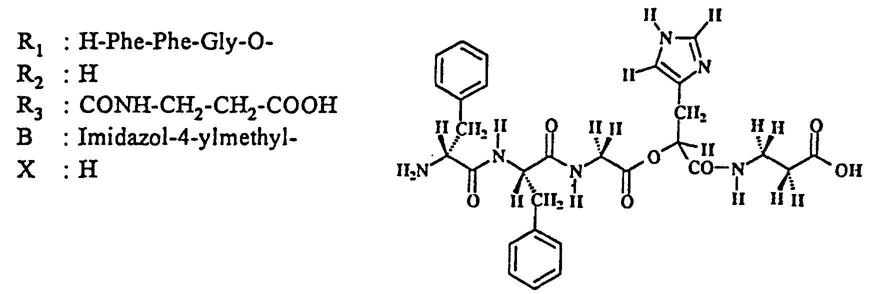

[2-(1H-(5-Methyl-imidazol-4-yl)-2-(phenylalanyl-phenylalanyl-glycyloxy)]-acetyl-β-alanin**(9a)**: (C₂₉H₃₄N₆O₇)
MS (FAB) : M/Z (3-NBA) = 712 ([M+Cs+H]^{⊕}), 579 ([M+H]^{⊕}).

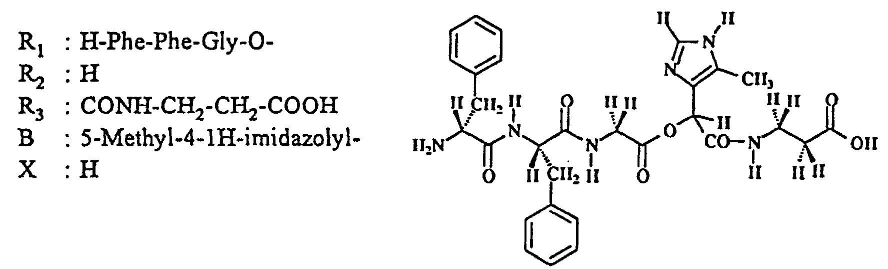

### [2-1H-Imidazol-4-yl-2-(phenylalanyl-phenylalanyl-glycyloxy)]-acetyl-β-alaninyl-carrier (10b):

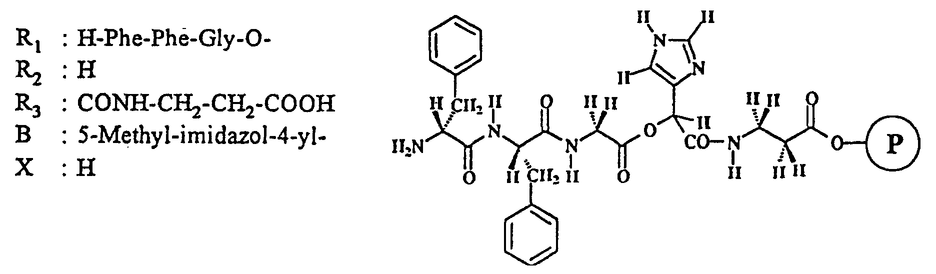

### [Phenylalanyl-phenylalanyl-glycyloxy)]-acetyl-β-alanin (11a) :

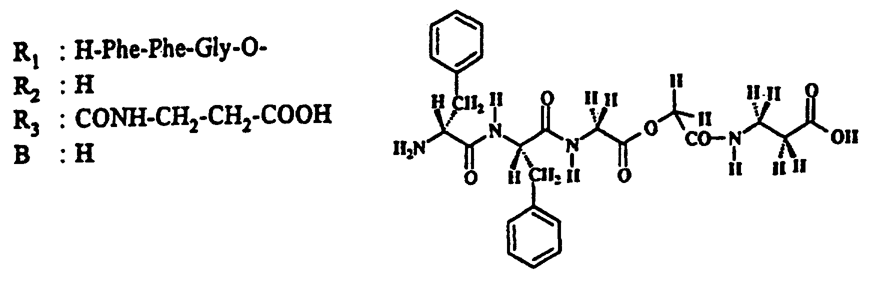

Das Schutzgruppenkonzept wurde am Beispiel der oben aufgeführten Verbindungen mit B = 1H-Imidazolyl und X = tert-Butyloxycarbonyl sowohl in Lösung als auch gebunden an einen festen Papierträger (Whatman 3MM) mit Hinblick auf die spezielle Verwendung als orthogonale Ankergruppe für die Festphasenpeptidsynthese überprüft.

### Test in Lösung

Die Verbindungen **(8)** und **(9)** wurden an einen p-Benzyloxybenzyl-derivatisierten Polystyrol-Harz ('Wang-Harz' der Firma Novabiochem) synthetisiert. Dazu wurde N^{β}-9-Fluorenylmethoxycarbonyl-β-alanin an das Harz durch Aktivierung der Carboxylfunktion mit Mesitylensulfonyl-3-nitro-1.2.4-triazol (MSNT) unter Katalyse von N-Methylimidazol (MeIm) in DCM verestert [Blankemeyer-Menge, B. und Frank, R, Tetrahedron Lett, 31, 1701 (1990)]. Nach Abspaltung der Aminoschutzgruppe mit einem Überschuß an 20% Piperidin in DMF wurde **(4)** bzw. **(6)** durch N.N-Diisopropylcarbodiimid (DIC) (1.2 eq.) / Hydroxybenzotriazol (HOBt) (2.0 eq.)-Aktivierung der Carboxyl-Funktion in DMF an die gebundene H-βAlanin-Einheit gekoppelt. Die schrittweise Synthese des Tripeptides H-Phe-Phe-Gly- begann mit der MSNT/MeIm-aktivierten Veresterung des N^{α}-Fmoc-Gly-OH an die Ankergruppe. Im weiteren wurde nach üblichen Peptidsynthesebedingungen unter Aktivierung von DIC/HOBt nach der N^{α}-Fmoc/tBu-Strategie verfahren [Fields, G.B. and Noble, R.L., Int. J. Peptide Protein Res. 35, 161-214 (1990)]. Alle Kopplungsschritte verliefen unter nahezu quantitativer Ausbeute (UV/VIS-Analytik des Dibenzofulven/Piperidin-Adduktes). Die Verbindungen **(8a)** und **(9a)** wurden vom festen Träger mit Trifluoressigsäure (TFA)/ Dichlormethan(DCM) (1:1) (2.5 % Triisobutylsilan (TIBS), 2.5 % Wasser) abgespalten und RP-C₁₈-chromatographisch aufgereinigt. Dabei wird gleichzeitig die Imidazolylschutzgruppe X = Boc abgespalten. Als direkten Vergleich zu den hier speziell beschriebenen Ankergruppen wurde die Verbindung H-Phe-Phe-Gly-O-Glycolsäure-βAla-OH **(11a)** nach dem oben beschriebenen Verfahren an fester Phase synthetisiert, wie oben abgespalten und durch RP-C₁₈-HPLC aufgereinigt. Die am Imidazolyl-Rest (B) entschützten, jetzt labil geschützten Verbindungen **(8a)**, **(9a)** sowie die Vergleichssubstanz **(11a)** wurden in Lösung auf die Labilität der Esterbindung gegenüber den unten aufgeführten wässrigen Puffern untersucht. Tabelle 1 gibt die Ergebnisse wieder.

### Verwendete Puffer :

(a) NaH₂PO₄/Na₂HPO₄/0.1M/pH 7.0/H₂O
(b) NaH₂PO₄/Na₂HPO₄/0.1M/pH 7.5/H₂O
(c) NaH₂PO₄/Na₂HPO₄/0.01M/pH 7.0/H₂O
(d) NaH₂PO₄/Na₂HPO₄/0.01M/pH 7.5/H₂O
(e) Na₃BO₄/H₃BO₄/0.05 M/pH 7.6/H₂O
(f) Na₃BO₄/H₃BO₄/0.05 M/pH 8.0/H₂O
(g) tris-Hydroxymethylaminomethan-hydrochlorid (Tris.HCl)/0.01 M/pH 7.6/H₂O
(h) tris-Hydroxymethylaminomethan-hydrochlorid (Tris.HCl)/0.01 M/pH 8.0/H₂O
(i) NaHCO₃/Na₂CO₃/0.05 M/pH 9.6 /H₂O
(j) NaHCO₃/Na₂CO₃/0.05 M/pH 10.0 /H₂O
(k) DMF/Wasser = 1:9 (pH 7.0)
(l) Triethylammoniumacetat (TEAAc)/0.01 M/pH 7.0/H₂O

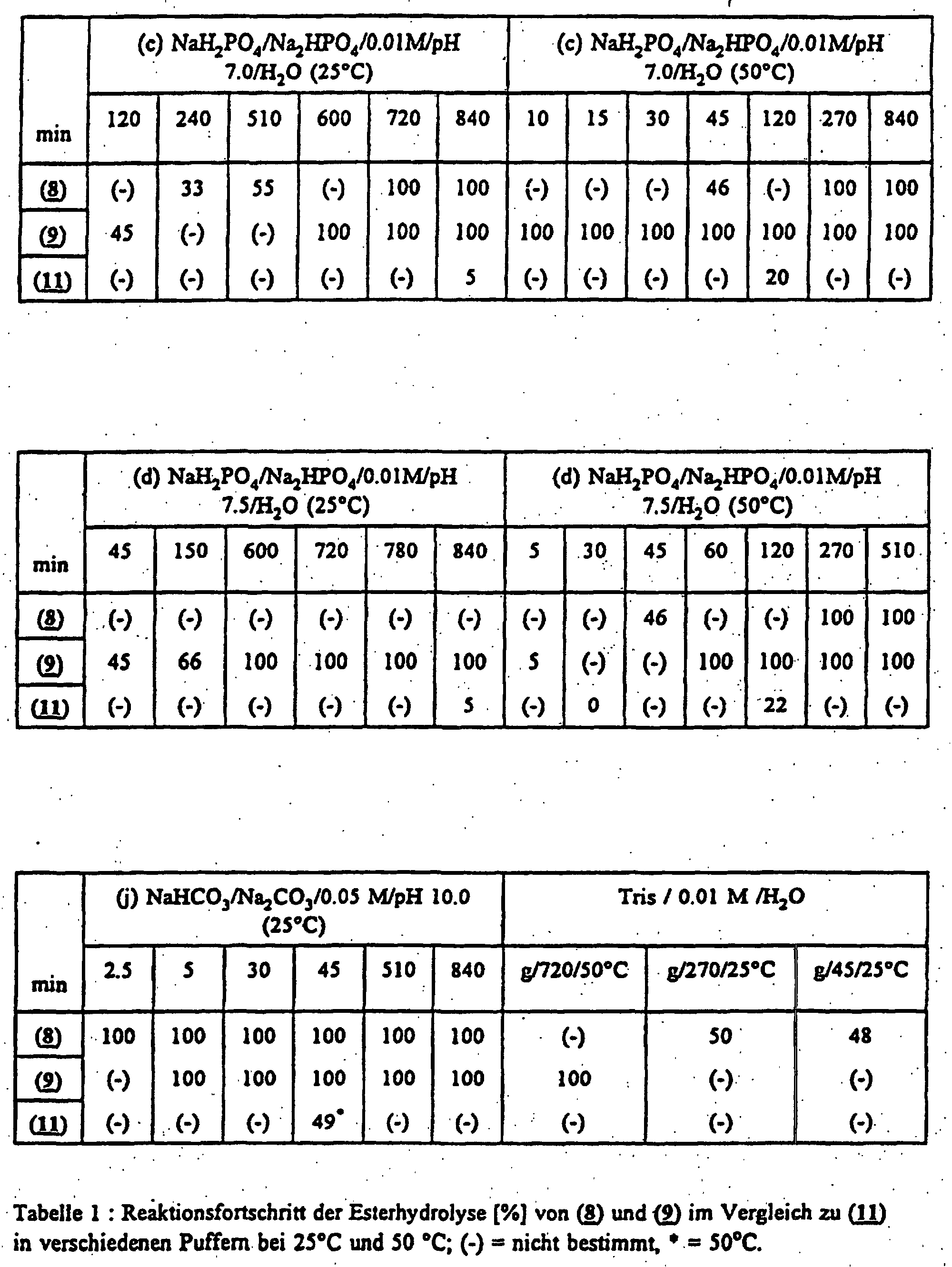

### Test am polymeren Träger

Die analogen, polymergebundenen Verbindungen zu **(8a)**, **(9a)** und **(11a)** wurden wie oben aber auf Papierrundfiltern (Whatman 3MM) synthetisiert. Die Behandlung mit TFA/DCM (2.5 % TIBS, 2.5 % H₂O) spaltet in diesem speziellen Fall nur die Imidazolylschutz-gruppe X = Boc ab, berührt jedoch die Esterbindung zwischen C-terminalen Carboxylfunktion und dem festen Träger nicht (polymergebundene Verbindungen werden im weiteren mit einem b gekennzeichnet). Zusätzlich zu **(8b)**, **(9b)** und **(11b)** wurde eine Verbindung **(10b)** synthetisiert die mit dem Ankermolekül **(5)** an die feste Phase gebunden war. Nach Abspalten der Schutzgruppe X vom polymergebundenen **(8b)**-**(10b)** wird der Filter 2 x 5 min mit dem Abspaltreagenz, 3 x 10 min mit 1 % HCl in Methanol/Wasser (1:1) und 3 x 10 min mit 1 M Essigsäure/Wasser gewaschen und im HV 12 h getrocknet. In den Waschlösungen ist kein Tripeptid H-Phe-Phe-Gly-OH nachzuweisen.

**Tabelle 2 :**

| Abspaltung des Tripeptides H-Phe-Phe-Gly-OH von den festphasengebundenen Verbindungen (**8b**)-(**11b**) in verschieden Puffern bei 50°C über 12 h. Die Meßfehler sind mit einem Fehler von ca. 20 % behaftet. | | | | | | | | | |
|---|---|---|---|---|---|---|---|---|---|
| | (a) | (b) | (c) | (d) | (e) | (f) | (h) | (k) | (l) |
| (**8b**) | 100 | 100 | 100 | 100 | 100 | 100 | 80 | 5 | 90 |
| (**9b**) | 100 | 100 | 100 | 100 | 100 | 100 | 90 | 5 | 60 |
| (**10b**) | 100 | 100 | 100 | 100 | 100 | 100 | 50 | 5 | 80 |
| (**11b**) | nd | 25 | nd | 25 | 20 | 40 | nd | 1 | 25 |

Die Ergebnisse zeigen deutlich,
- daß die mit X geschützte Schutzgruppe unter den basischen Reaktionsbedingungen (z.B. 20 % Piperidin in DMF) der Synthese von R₁ stabil ist
- daß die Esterbindung der Schutzgruppe an die Carboxylverbindung unter sauren wäßrigen Bedingungen stabil ist und die entsprechend geschützten Verbindungen gereinigt werden können
- daß die basische Funktion des Typs von Schutz- bzw. Ankergruppe zu einer drastisch erhöhten Hydrolyserate im Vergleich zu den nicht substituierten Glycolsäurederivaten führt.
- daß eine Abspaltung der Schutzgruppe (ohne X) auch unter neutralen Reaktionsbedingungen (pH = 7) möglich ist.

3-(N^{im}-tert-Butoxycarbonyl-imidazol-4-yl)-2-bromo-propionsäure **(12)** : (C₁₁H₁₅BrN₂O₄) Die Synthese erfolgte analog zu (**4**) ausgehend von 3-1H-Imidazol-4-yl-2-bromo-propionsäure (z.B. kommerziell erhältlich bei Sigma). Ausb. 67 % d. Th. 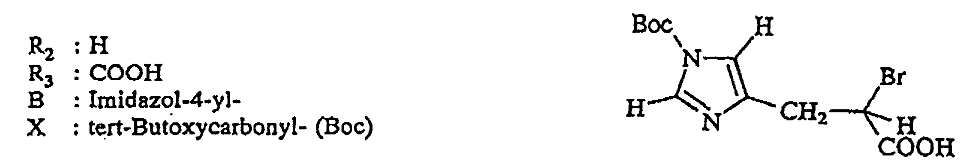 und Hydroxylfunktion (-OH) ersetzt durch Brom-Funktion (-Br)
¹H-NMR (400 MHz, CDCl₃) : δ = 8.15 (s, 1H, 2-H), 7.27 (s, 1H, 4-H), 5.55 (s, 1H, CHBr), 1.58 (s, 9H, CH₃).- ¹³C-NMR (75 MHz, CDCl₃) : δ = 176.1 (s,COOH), 146.3 (s, N-COO-), 136.8 (d, C-2), 135.9 (s, C-5), 116.1 (d, C-4), 86.4 (s, C(CH₃)₃), 73.5 (d, CHOH), 27.8 (q, CH₃).

### Verwendete Abkürzungen :

Aminosäurederivate nach IUPAC-IUB [J. Biol. Chem. 260. 14(1983)]
- Boc: tert.-Butyloxycarbonyl
- DCHA: Dicyclohexylammonium
- DCM: Dichlormethan
- DIC: N,N'-Diisopropylcarbodiimid
- DMF: Dimethylformamid
- DMSO: Dimethylsulfoxid
- FAB-MS: "Fast Atom Bombardement" Massenspektroskopie
- Fmoc: 9-Fluorenylmethoxycarbonyl
- Hal: Halogen
- HOBt: N-Hydroxybenzotriazol
- HPLC: Hochdruckflüssigchromatographie
- HV: Hochvakuum
- MeIm: N-Methylimidazol
- MSNT: Mesitylensulfonyl-3-nitro-1.2.4-triazol
- 3-NBA: 3-Nitrobenzylalkohol
- NMR: Kemresonanzspektroskopie
- TIBS: Triisobutylsilan
- TFA: Trifluoressigsäure

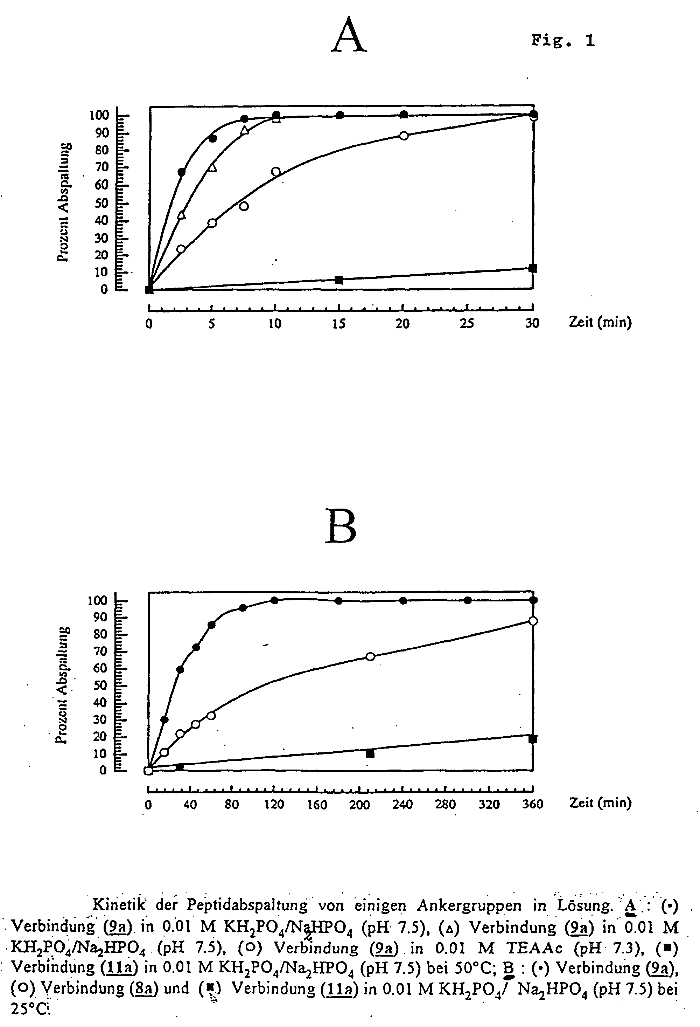

## Patentansprüche

1. Peptidsynthese, bei der man
(i) eine Verbindung der Formel
Y-C(R₂)(R₃)-B-X
wobei
Y = HO, Cl, Br oder I,
B = aromatischer stickstoffhaltiger Fünfring,
X = Schutzgruppe für B, die die Basizität von B soweit herabsetzt, daß die intramolekulare Hydrolyse einer Esterbindung erst nach Abspaltung von X erfolgen kann,
R₂ = H, Alkyl oder Aryl, und
R₃ = HOOC; oder Alkyl oder Aryl, jeweils durch eine reaktive Funktion substituiert, die eine Ankergruppe bildet,
mit einem Trägermaterial verknüpft,
(ii) danach die mit dem Träger verknüpfte Verbindung mit einer Carboxyl-Funktion (COOH) unter Ausbildung einer Esterbindung eines Esters eines Mono-, Di- oder Oligopeptids umsetzt und ein Peptid synthetisiert,
(iii) danach die Schutzgruppe X selektiv abspaltet,
(iv) danach das angefallene Zwischenprodukt reinigt und
(v) danach das angefallene Zwischenprodukt in wässeriger Lösung zerfallen lässt und das Peptid als Syntheseprodukt mit freier Carboxyl-Funktion gewinnt.

2. Peptidsynthese, bei der man
(i) eine Verbindung der Formel
Y-C(R₂)(R₃)-B-X
wobei
Y = HO, Cl, Br oder I,
B = gegebenenfalls im Ring methylsubstituiertes Imidazol-yl oder gegebenenfalls im Ring methylsubstituiertes Imidazol-yl-methylen,
X = Schutzgruppe für B, die die Basizität von B soweit herabsetzt, daß die intramolekulare Hydrolyse der Esterbindung erst nach Abspaltung von X erfolgen kann,
R₂ = H, Alkyl oder Aryl, und
R₃ = HOOC; oder Alkyl oder Aryl, jeweils durch eine reaktive Funktion substituiert, die eine Ankergruppe bildet,
mit einem Trägermaterial verknüpft,
(ii) danach die mit dem Träger verknüpfte Verbindung mit einer Carboxyl-Funktion (COOH) unter Ausbildung einer Esterbindung eines Esters eines Mono-, Di- oder Oligopeptids umsetzt und ein Peptid synthetisiert,
(iii) danach die Schutzgruppe X selektiv abspaltet,
(iv) danach das angefallene Zwischenprodukt reinigt und
(v) danach das angefallene Zwischenprodukt in wässeriger Lösung zerfallen lässt und das Peptid als Syntheseprodukt mit freier Carboxyl-Funktion gewinnt.

3. Peptidsynthese nach Anspruch 1 oder 2, **dadurch gekennzeichnet, dass** man Stufe (v) in physiologischer Pufferlösung durchführt.

4. Peptidsynthese nach Anspruch 1 oder 2 oder 3, **dadurch gekennzeichnet, dass** man die Peptidsynthese bei Stufe (ii) unter basischen Reaktionsbedingungen durchführt.

5. Peptidsynthese nach einem der vorhergehenden Ansprüche, **dadurch gekennzeichnet, dass** in der Verbindung der Formel gemäß Anspruch 1 oder 2 R₃ durch H₂N, HS, OH oder COOH substituiert ist.

6. Peptidsynthese nach einem der vorhergehenden Ansprüche, **dadurch gekennzeichnet, dass** man eine Verbindung der Formel gemäß Anspruch 1 oder 2 verwendet mit R₃ = HOOC-C₁₋₁₂-alkyl, HOOC-phenyl, HO-C₁₋₁₂-alkyl, HO-phenyl, H₂N-C₁₋₁₂-alkyl, H₂N-phenyl, HS-C₁₋₁₂-alkyl oder HS-phenyl.

7. Peptidsynthese nach einem der vorhergehenden Ansprüche, **dadurch gekennzeichnet, daß** man eine Verbindung der Formel gemäß Anspruch 1 oder 2 verwendet mit X = Urethan- oder Alkoxycarbonyl Schutzgruppe.

8. Peptidsynthese nach Anspruch 7, **dadurch gekennzeichnet, dass** man eine Verbindung der Formel gemäß Anspruch 1 oder 2 verwendet mit X = tert-Butoxycarbonyl (Boc).

9. Peptidsynthese nach einem der vorhergehenden Ansprüche, **dadurch gekennzeichnet, daß** man eine Verbindung der Formel gemäß Anspruch 1 oder 2 verwendet mit R₂ = C₁₋₁₂-Alkyl oder Phenyl.

10. Peptidsynthese nach einem der Ansprüche 1 und 3 bis 9, **dadurch gekennzeichnet, daß** man eine Verbindung der Formel gemäß Anspruch 1 und 2 verwendet mit B = Imidazol-yl.

11. Peptidsynthese nach Anspruch 10, **dadurch gekennzeichnet, daß** man eine Verbindung der Formel gemäß Anspruch 1 verwendet mit B = Imidazol-4-yl oder Imidazol-2-yl.

12. Peptidsynthese nach einem der Ansprüche 2 bis 9, **dadurch gekennzeichnet, daß** man eine Verbindung der Formel gemäß Anspruch 2 verwendet mit B = Imidazol-4-yl-methylen oder 5-Methyl-imidazol-4-yl.

## Claims

1. Peptide synthesis, wherein
(i) a compound of the formula
Y-C(R₂)(R₃)-B-X,
where
Y = HO, Cl, Br or I,
B = an aromatic nitrogen-containing five-membered ring,
X = a protective group for B which reduces the basicity of B to such a degree that the intramolecular hydrolysis of an ester bond cannot happen before a splitting off of X,
R₂ = H, alkyl or aryl, and
R₃ = HOOC; or alkyl or aryl, each substituted by a reactive function which forms an anchor group,
is linked to a carrier material,
(ii) thereafter the compound linked to the carrier is reacted with a carboxylic function (COOH) by forming an ester bond of an ester of a mono, di or oligopeptide and a peptide is synthesized,
(iii) thereafter the protective group X is selectively split off,
(iv) thereafter the formed intermediate product is purified, and
(v) thereafter the formed intermediate is allowed to decompose in aqueous solution and the peptide is recovered as synthesis product with free carboxylic function.

2. Peptide synthesis, wherein
(i) a compound of the formula
Y-C(R₂)(R₃)-B-X,
where
Y = HO, Cl, Br or I,
B = imidazol-yl which is optionally methyl-substituted in the ring, or imidazol-yl-methylene which is optionally methyl-substituted in the ring,
X = a protective group for B which reduces the basicity of B to such a degree that the intramolecular hydrolysis of an ester bond cannot happen before a splitting off of X,
R₂ = H, alkyl or aryl, and
R₃ = HOOC; or alkyl or aryl, each substituted by a reactive function which forms an anchor group,
is linked to a carrier material,
(ii) thereafter the compound linked to the carrier is reacted with a carboxylic function (COOH) by forming an ester bond of an ester of a mono, di or oligopeptide and a peptide is synthesized,
(iii) thereafter the protective group X is selectively split off,
(iv) thereafter the formed intermediate product is purified, and
(v) thereafter the formed intermediate product is allowed to decompose in aqueous solution and the peptide is recovered as synthesis product with free carboxylic function.

3. Peptide synthesis according to claim 1 or 2, **characterized in that** step (v) is carried out in a physiological puffer solution.

4. Peptide synthesis according to claim 1 or 2 or 3, **characterized in that** the peptide synthesis at step (ii) is carried out under basic reaction conditions.

5. Peptide synthesis according to any of the preceding claims, **characterized in that** R₃ of the compound of the formula according to claim 1 or 2 is substituted by H₂N, HS, OH or COOH.

6. Peptide synthesis according to any of the preceding claims, **characterized in that** a compound of the formula according to claim 1 or 2 is used wherein R₃ means HOOC-C₁₋₁₂-alkyl, HOOC-phenyl, HO-C₁₋₁₂-alkyl, HO-phenyl, H₂N-C₁₋₁₂-alkyl, H₂N-phenyl, HS-C₁₋₁₂-alkyl or HS-phenyl.

7. Peptide synthesis according to any of the preceding claims, **characterized in that** a compound of the formula according to claim 1 or 2 is used wherein X means a urethane or an alkoxycarbonyl protective group.

8. Peptide synthesis according to claim 7, **characterized in that** a compound of the formula according to claim 1 or 2 is used wherein X means tert-butoxycarbonyl (Boc).

9. Peptide synthesis according to any of the preceding claims, **characterized in that** a compound of the formula according to claim 1 or 2 is used wherein R₂ means C₁₋₁₂-alkyl or phenyl.

10. Peptide synthesis according to any of claims 1 and 3 to 9, **characterized in that** a compound of the formula according to claim 1 or 2 is used wherein B means imidazol-yl.

11. Peptide synthesis according to claim 10, **characterized in that** a compound of the formula according to claim 1 is used wherein B means imidazol-4-yl or imidazol-2-yl.

12. Peptide synthesis according to any of claims 2 to 9, **characterized in that** a compound of the formula according to claim 2 is used wherein B means imidazol-4-yl-methylen or 5-methyl-imidazol-4-yl.

## Revendications

1. Synthèse de peptides, au cours de laquelle
i) on fixe sur un matériau support un composé de formule
Y-C(R₂)(R₃)-B-X
dans laquelle
Y représente OH, Cl, Br ou I,
B représente un cycle aromatique azoté à cinq chaînons,
X représente un groupe protecteur de B, qui amoindrit la basicité de B à tel point que l'hydrolyse intramoléculaire d'une liaison ester ne peut se produire qu'après élimination de X,
R₂ représente un atome d'hydrogène ou un groupe alkyle ou aryle,
et R₃ représente COOH, ou un groupe alkyle ou aryle portant comme substituant, dans chaque cas, un groupe fonctionnel réactif constituant un groupe d'ancrage ;
ii) puis on fait réagir le composé fixé au support avec un groupe fonctionnel carboxyle COOH, avec formation de la liaison ester d'un ester de monopeptide, de dipeptide ou d'oligopeptide, et l'on synthétise un peptide ;
iii) puis on élimine sélectivement le groupe protecteur X ;
iv) puis on purifie le produit intermédiaire formé ;
v) puis on fait se décomposer, en solution aqueuse, le produit intermédiaire formé, et l'on obtient, en guise de produit de synthèse, un peptide à groupe carboxyle libre.

2. Synthèse de peptides, au cours de laquelle
i) on fixe sur un matériau support un composé de formule
Y-C(R₂)(R₃)-B-X
dans laquelle
Y représente OH, Cl, Br ou I,
B représente un groupe imidazolyle dont le cycle porte éventuellement un substituant méthyle, ou un groupe imidazolyl-méthylène dont le cycle porte éventuellement un substituant méthyle,
X représente un groupe protecteur de B, qui amoindrit la basicité de B à tel point que l'hydrolyse intramoléculaire d'une liaison ester ne peut se produire qu'après élimination de X,
R₂ représente un atome d'hydrogène ou un groupe alkyle ou aryle,
et R₃ représente COOH, ou un groupe alkyle ou aryle portant comme substituant, dans chaque cas, un groupe fonctionnel réactif constituant un groupe d'ancrage ;
ii) puis on fait réagir le composé fixé au support avec un groupe fonctionnel carboxyle COOH, avec formation de la liaison ester d'un ester de monopeptide, de dipeptide ou d'oligopeptide, et l'on synthétise un peptide ;
iii) puis on élimine sélectivement le groupe protecteur X ;
iv) puis on purifie le produit intermédiaire formé ;
v) puis on fait se décomposer, en solution aqueuse, le produit intermédiaire formé, et l'on obtient, en guise de produit de synthèse, un peptide à groupe carboxyle libre.

3. Synthèse de peptides conforme à la revendication 1 ou 2, **caractérisée en ce que** l'on effectue l'étape (v) dans de la solution tampon physiologique.

4. Synthèse de peptides conforme à la revendication 1 ou 2 ou 3, **caractérisée en ce que** c'est en milieu basique que l'on effectue la synthèse d'un peptide, lors de l'étape (ii).

5. Synthèse de peptides conforme à l'une des revendications précédentes, **caractérisée en ce que**, dans le composé de formule donnée dans la revendication 1 ou 2, R₃ porte en tant que substituant un groupe NH₂, SH, OH ou COOH.

6. Synthèse de peptides conforme à l'une des revendications précédentes, **caractérisée en ce que** l'on utilise un composé de formule donnée dans la revendication 1 ou 2, dans laquelle R₃ représente un groupe HOOC-(alkyle en C₁₋₁₂), HOOC-phényle, HO-(alkyle en C₁₋₁₂), HO-phényle, H₂N-(alkyle en C₁₋₁₂), H₂N-phényle, HS-(alkyle en C₁₋₁₂) ou HS-phényle,

7. Synthèse de peptides conforme à l'une des revendications précédentes, **caractérisée en ce que** l'on utilise un composé de formule donnée dans la revendication 1 ou 2, dans laquelle X représente un groupe protecteur de type uréthane ou alcoxycarbonyle.

8. Synthèse de peptides conforme à la revendication 7,
**caractérisée en ce que** l'on utilise un composé de formule donnée dans la revendication 1 ou 2, dans laquelle X représente un groupe tertio-butoxycarbonyle (Boc).

9. Synthèse de peptides conforme à l'une des revendications précédentes, **caractérisée en ce que** l'on utilise un composé de formule donnée dans la revendication 1 ou 2, dans laquelle R₂ représente un groupe alkyle en C₁₋₁₂ ou phényle.

10. Synthèse de peptides conforme à l'une des revendications 1 et 3 à 9, **caractérisée en ce que** l'on utilise un composé de formule donnée dans la revendication 1, dans laquelle B représente un groupe imidazolyle.

11. Synthèse de peptides conforme à la revendication 10, **caractérisée en ce que** l'on utilise un composé de formule donnée dans la revendication 1, dans laquelle B représente un groupe imidazol-4-yle ou imidazol-2-yle.

12. Synthèse de peptides conforme à l'une des revendications 2 à 9, **caractérisée en ce que** l'on utilise un composé de formule donnée dans la revendication 2, dans laquelle B représente un groupe imidazol-4-yl-méthylène ou 5-méthyl-imidazol-4-yle.
